# EUROPEAN PATENT SPECIFICATION

(11) **EP 1 767 542 B1**
(45) Date of publication and mention of the grant of the patent: **11.05.2016**
(21) Application number: 05075377.1
(22) Date of filing: 21.03.1997
(51) Int. Cl.: C07K 7/00, C12N 15/12, C12N 15/33, C07K 14/18, C07K 14/02, C07K 14/16

(54) **HLA-A2.1 binding peptides and their uses**
HLA-A2.1 bindende Peptide und deren Verwendung
Peptides de fixation de HLA-A2.1 et leurs utilisation

(30) Priority: 21.03.1996 US 13980 P; 20.03.1997 US 822382
(43) Date of publication of application: 28.03.2007
(62) Divisional of application: 97918494.2
(73) Proprietor: Epimmune Inc., San Diego, CA 92121 (US)
(72) Inventor: Grey, Howard M, La Jolla, CA 92037 (US); Sette, Alessandro, La Jolla, CA 92037 (US); Sidney, John, San Diego, CA 92130 (US)
(74) Representative: Inspicos P/S

(56) References cited:
- WO-A-91/00912
- WO-A-94/20127
- WO-A-95/28958
- WO-A-96/18409
- WO-A1-94/25486
- WO-A1-94/25874
- WO-A1-95/25122
- DATABASE Geneseq [Online] 26 May 1995 (1995-05-26), "KHCV E2E envelope protein antigen.", XP002684732, retrieved from EBI accession no. GSP:AAR62514 Database accession no. AAR62514
- BURG VAN DER S H ET AL: "INDUCTION OF A PRIMARY HUMAN CYTOTOXIC T-LYMPHOCYTE RESPONSE AGAINST A NOVEL CONSERVED EPITOPE IN A FUNCTIONAL SEQUENCE OF HIV -1 REVERSE TRNSCRIPTASE", CURRENT SCIENCE, vol. 9, no. 2, 1 February 1995 (1995-02-01), pages 121-127, XP000770744, ISSN: 0011-3891

## Description

### BACKGROUND OF THE INVENTION

The present invention relates to compositions and methods for preventing, treating or diagnosing a number of pathological states such as viral diseases and cancers. In particular, it provides novel peptides capable of binding selected major histocompatibility complex (MHC) molecules and inducing an immune response.

MHG molecules are classified as either Class I or Class II molecules. Class II MHC molecules are expressed primarily on cells involved in initiating and sustaining immune responses, such as T lymphocytes, B lymphocytes, macrophages, etc. Class II MHC molecules are recognized by helper T lymphocytes and induce proliferation of helper T lymphocytes and amplification of the immune response to the particular immunogenic peptide that is displayed. Class I MHC molecules are expressed on almost all nucleated cells and are recognized by cytotoxic T lymphocytes (CTLs), which then destroy the antigen-bearing cells. CTLs are particularly important in tumor rejection and in fighting viral infections.

The CTL recognizes the antigen in the form of a peptide fragment bound to the MHC class I molecules rather than the intact foreign antigen itself. The antigen must normally be endogenously synthesized by the cell, and a portion of the protein antigen is degraded into small peptide fragments in the cytoplasm. Some of these small peptides translocate into a pre-Golgi compartment and interact with class I heavy chains to facilitate proper folding and association with the subunit β2 microglobulin. The peptide-MHC class I complex is then routed to the cell surface for expression and potential recognition by specific CTLs.

Investigations of the crystal structure of the human MHC class I molecule, HLA-A2.1, indicate that a peptide binding groove is created by the folding of the α1 and a2 domains of the class I heavy chain (Bjorkman et al., Nature 329:506 (1987). In these investigations, however, the identity of peptides bound to the groove was not determined.

Buus et al., Science 242:1065 (1988) first described a method for acid elution of bound peptides from MHC. Subsequently, Rammensee and his coworkers (Falk et al., Nature 351:290 (1991) have developed an approach to characterize naturally processed peptides bound to class I molecules. Other investigators have successfully achieved direct amino acid sequencing of the more abundant peptides in various HPLC fractions by conventional automated sequencing of peptides eluted from class I molecules of the B type (Jardetzky, et al., Nature 353:326 (1991) and of the A2.1 type by mass spectrometry (Hunt, et al., Science 225:1261 (1992). A review of the characterization of naturally processed peptides in MHC Class I has been presented by Rötzschke and Falk (Rötzschke and Falk, Immunol. Today 12:447 (1991).

Sette et al., Proc. Natl. Acad. Sci. USA 86:3296 (1989) showed that MHC allele specific motifs could be used to predict MHC binding capacity. Schaeffer et al., Proc. Natl. Acad. Sci USA 86:4649 (1989) showed that MHC binding was related to immunogenicity. Several authors (De Bruijn et al., Eur. J. Immunol., 21:2963-2970 (1991); Pamer et al., 991 Nature 353:852-955 (1991)) have provided preliminary evidence that class I binding motifs can be applied to the identification of potential immunogenic peptides in animal models. Class I motifs specific for a number of human alleles of a given class I isotype have yet to be described. It is desirable that the combined frequencies of these different alleles should be high enough to cover a large fraction or perhaps the majority of the human outbred population.

WO 94/25486 A1 and WO 94/25874 A1 relate to diagnostic methods for hepatitis C virus (HCV) infection and disclose amino acid sequences of HCV proteins, among them a fragment designated E2E. A related sequence is also disclosed in Geneseq database No. XP002684732 (retrieved from EBI accession no. GSP:AAR62514).

Despite the developments in the art, the prior art has yet to provide a useful human peptide-based vaccine or therapeutic agent based on this work. The present invention provides these and other advantages.

### SUMMARY OF THE INVENTION

The present invention provides immunogenic peptides having binding motifs for HLA-A2.1 molecules and bind to the appropriateMHC allele.

In particular, the present invention provides
(1) An isolated peptide of less than 15 amino acids, which comprises the amino acid sequence LLFLLLADA (SEQ I D NO:2).
(2) The isolated peptide of (1), wherein the peptide consists of the amino acid sequence LLFLLLADA (SEQ ID NO:2).
(3) The isolated peptide of (1) or (2), wherein the peptide is linked to another peptide.
(4) The isolated peptide of (1) or (2), wherein the peptide is linked to a second molecule.
(5) The isolated peptide of (4), wherein the second molecule is a lipid, a T helper epitope, a cytotoxic T lymphocyte (CTL) epitope, or a carrier molecule.
(6) A composition which comprises an isolated peptide of any one of (1) to (5) complexed with an HLA A2.1 molecule present on an antigen-presenting cell.
(7) A pharmaceutical composition which comprises a peptide of any one of (1) to (5) and a pharmaceutically acceptable excipient.
(8) An isolated nucleic acid for use in the treatment or prevention of HCV infection, which nucleic acid comprises a nucleotide sequence encoding a polypeptide that comprises CTL epitopes from HCV joined to one another, wherein one or more epitopes comprise the amino acid sequence LLFLLLADA.
(9) An isolated nucleic acid which comprises a nucleotide sequence encoding a polypeptide that comprises CTL epitopes from HCV joined to one another, wherein one or more epitopes comprise the amino acid sequence LLFLLLADA, and wherein one or more further epitopes consists of the amino acid sequence: ILSPGALVV (SEQ ID NO:1); LLFLLLADA (SEQ I D NO:2); WMNRLIAFA (SEQ ID NO:3); or VLVGGVLAA (SEQ I D NO:4).
(10) The isolated nucleic acid of (9), wherein the polypeptide comprises further epitopes consisting of the amino acid sequences ILSPGALVV (SEQ ID NO:1), LLFLLLADA (SEQ ID NO:2), WMNRLIAFA (SEQ ID NO:3), and VLVGGVLAA (SEQ ID NO:4).
(11) The isolated nucleic acid of (9), wherein the further epitopes consist of the amino acid sequences: YMDDVVLGV (SEQ ID NO:29), FLPSZFFPSV (SEQ ID NO:30), FLPSDAFPSV (SEQ ID NO:31), FLPSAFFPSV (SEQ ID NO:32), FLPSDFAPSV (SEQ ID NO:33), FLPSDFFASV (SEQ ID NO:34), FLPSDFFPAV (SEQ ID NO:35), FLASDFFPSV (SEQ ID NO:36), ALPSDFFPSV (SEQ ID NO:37), YLPSDFFPSV (SEQ ID NO:38), FMPSDFFPSV (SEQ ID NO:39), FLKSDFFPSV (SEQ ID NO:40), FLPSEFFPSV (SEQ ID NO:41), FLPSDFYPSV (SEQ ID NO:42), FLPSDFFKSV (SEQ ID NO:43), and FLPSDFFPKV (SEQ ID NO:44).
(12) The isolated nucleic acid of any one of (9) to (11), wherein the polypeptide comprises flanking sequences adjacent to one or more of the CTL epitopes.
(13) A polypeptide encoded by the nucleic acid of any one of (9) to (12).
(14) A pharmaceutical composition comprising an isolated nucleic acid of any one of (9) to (12) and a pharmaceutically acceptable excipient.
(15) An *ex vivo* method for using an immunogenic peptide composition, which comprises providing an isolated peptide of less than 15 amino acids that comprises the amino acid sequence: LLFLLLADA (SEQ ID NO:2); complexing the immunogenic peptide with an HLA A2.1 molecule; and contacting in vitro an HLA A2.1-restricted CTL with the complex of the peptide and the HLA A2.1 molecule, whereby a CTL response is induced.
(16) The method of (15), wherein the peptide consists of the amino acid sequence: LLFLLLADA (SEQ ID NO:2).
(17) The use of an immunogenic peptide for the manufacture of a medicament for the treatment or prevention of HCV infection, wherein the peptide is less than 15 amino acids and comprises the amino sequence LLFLLLADA (SEQ ID NO:2).
(18) The use of (17), wherein the peptide consists of the amino acid sequence LLFLLLADA (SEQ ID NO:2).
(19) The use of a nucleic acid for the manufacture of a medicament for the treatment or prevention of HCV infection, wherein the nucleic acid comprises a nucleotide sequence encoding a polypeptide that comprises CTL epitopes from HCV joined to one another, wherein one or more epitopes comprise the amino acid sequence LLFLLLADA (SEQ ID NO:2).
(20) The use of (19), wherein one of the epitopes consists of the amino acid sequence LLFLLLADA (SEQ ID NO:2).
(21) The use of (19), wherein the polypeptide comprises further epitopes consisting of the amino acid sequences: ILSPGALVV (SEQ ID NO:1), LLFLLLADA (SEQ ID NO:2), WMNRLIAFA (SEQ ID NO:3), and VLVGGVLAA (SEQ ID NO:4).
(22) The use of any one of (19) to (21), wherein the polypeptide comprises flanking sequences adjacent to one or more of the CTL epitopes.
(23) The method of (15) or (16) wherein the HLA A2.1 molecule is expressed on a cell.

The peptides are based on sequences of antigenic proteins from hepatitis C. The peptides, or nucleic acids encoding them, are thus useful in pharmaceutical compositions for both in vivo and ex vivo therapeutic and diagnostic applications.

### Definitions

The term "peptide" is used interchangeably with "oligopeptide" in the present specification to designate a series of residues, typically L-amino acids, connected one to the other typically by peptide bonds between the alpha-amino and carbonyl groups of adjacent amino acids. The oligopeptides of the invention are less than about 15 residues in length and usually consist of between about 8 and about 11 residues, preferably 9 or 10 residues.

An "immunogenic peptide" is a peptide which comprises an allele-specific motif such that the peptide will bind an MHC molecule and induce a CTL response. Immunogenic peptides of the invention are capable of binding to an appropriate HLA-A2.1 molecule and inducing a cytotoxic T cell response against the antigen from which the immunogenic peptide is derived.

Immunogenic peptides are conveniently identified using the algorithms disclosed. The algorithms are mathematical procedures that produce a score which enables the selection of immunogenic peptides. Typically one uses the algorithmic score with a "binding threshold' to enable selection of peptides that have a high probability of binding at a certain affinity and will in turn be immunogenic. The algorithm is based upon either the effects on MHC binding of a particular amino acid at a particular position of a peptide or the effects on binding of a particular substitution in a motif containing peptide.

The relationship between binding affinity for MHC class I molecules and immunogenicity of discrete peptide epitopes has been analyzed in two different experimental approaches (Sette, et al., J. Immunol., 153:5586-5592 (1994)). In the first approach, the immunogenicity of potential epitopes ranging in MHC binding affinity over a 10,000-fold range was analyzed in HLA-A0201 transgenic mice. In the second approach, the antigenicity of approximately 100 different hepatitis B virus (HBV)-derived potential epitopes, all carrying A 0201 binding motifs, was assessed by using PBL of acute hepatits patients. In both cases, it was found that an affinity threshold of approximately 500 nM (preferably 500 nM or less) determines the capacity of a peptide epitope to elicit a CTL response. These data correlate well with class I binding affinity measurements of either naturally processed peptides or previously described T cell epitopes. These data indicate the important role of determinant selection in the shaping of T cell responses.

A "conserved residue" is an amino acid which occurs in a significantly higher frequency than would be expected by random distribution at a particular position in a peptide. Typically a conserved residue is one where the MHC structure may provide a contact point with the immunogenic peptide. One to three, preferably two, conserved residues within a peptide of defined length defines a motif for an immunogenic peptide. These residues are typically in close contact with the peptide binding groove, with their side chains buried in specific pockets of the groove itself. Typically, an immunogenic peptide will comprise up to three conserved residues, more usually two conserved residues.

As used herein, "negative binding residues" are amino acids which if present at certain positions (for example, positions 1, 3 and/or 7 of a 9-mer) will result in a peptide being a nonbinder or poor binder and in turn fail to be immunogenic i.e. induce a CTL response.

The term "motif" refers to the pattern of residues in a peptide of defined length, usually about 8 to about 11 amino acids, which is recognized by a particular MHC allele. The peptide motifs are typically different for each human MHC allele and differ in the pattern of the highly conserved residues and negative residues.

The binding motif for an allele can be defined with increasing degrees of precision. In one case, all of the conserved residues are present in the correct positions in a peptide and there are no negative residues in positions 1,3 and/or 7.

The phrases "isolated" or "biologically pure" refer to material which is substantially or essentially free from components which normally accompany it as found in its native state. Thus, the peptides of this invention do not contain materials normally associated with their in situ environment, e.g., MHC I molecules on antigen presenting cells. Even where a protein has been isolated to a homogenous or dominant band, there are trace contaminants in the range of 5-10% of native protein which co-purify with the desired protein. Isolated peptides of this invention do not contain such endogenous co-purified protein.

The term "residue" refers to an amino acid or amino acid mimetic incorporated in an oligopeptide by an amide bond or amide bond mimetic.

### DESCRIPTION OF THE PREFERRED EMBODIMENTS

The present invention relates to the determination of allele-specific peptide motifs for human Class I MHC (sometimes referred to as HLA) allele subtypes, in particular, peptide motifs recognized by HLA-A2.1 alleles. These motifs are then used to define T cell epitopes from the desired antigen, particularly those associated with HCV, for which the amino acid sequence of the potential antigen or autoantigen targets is known.

Epitopes on a number of potential target proteins can be identified in this manner. Examples of suitable antigens include prostate specific antigen (PSA), hepatitis B core and surface antigens (HBvc, HBVs) hepatitis C antigens, Epstein-Barr virus antigens, melanoma antigens (e.g., MAGE-1), human immunodeficiency virus (HIV) antigens and human papilloma virus (HPV) antigens.

Peptides comprising the epitopes from these antigens are synthesized and then tested for their ability to bind to the appropriate MHC molecules in assays using, for example, purified class I molecules and radioiodonated peptides and/or cells expressing empty class I molecules by, for instance, immunofluorescent staining and flow microfluorometry, peptide-dependent class I assembly assays, and inhibition of CTL recognition by peptide competition. Those peptides that bind to the class I molecule are further evaluated for their ability to serve as targets for CTLs derived from infected or immunized individuals, as well as for their capacity to induce primary in vitro or in vivo CTL responses that can give rise to CTL populations capable of reacting with virally infected target cells or tumor cells as potential therapeutic agents.

The MHC class I antigens are encoded by the HLA-A, B, and C loci. HLA-A and B antigens are expressed at the cell surface at approximately equal densities, whereas the expression of HLA-C is significantly lower (perhaps as much as 10-fold lower). Each of these loci have a number of alleles. The peptide binding motifs are relatively specific for each allelic subtype.

For peptide-based vaccines, the peptides of the present invention preferably comprise a motif recognized by an MHC I molecule having a wide distribution in the human population. Since the MHC alleles occur at different frequencies within different ethnic groups and races, the choice of target MHC allele may depend upon the target population. Table 1 shows the frequency of various alleles at the HLA-A locus products among different races. For instance, the majority of the Caucasoid population can be covered by peptides which bind to four HLA-A allele subtypes, specifically HLA-A2.1, Al, A3.2, and A24.1. Similarly, the majority of the Asian population is encompassed with the addition of peptides binding to a fifth allele HLA-A 11.2.

**TABLE 1**

| A Allele/Subtype | N(69)° | A(54) | C(502) |
|---|---|---|---|
| A1 | 10.1(7) | 1.8(1) | 27.4(138) |
| A2.1 | 11.5(8) | 37.0(20) | 39.8(199) |
| A2.2 | 10.1(7) | 0 | 3.3(17) |
| A2.3 | 1.4(1) | 5.5(3) | 0.8(4) |
| A2.4 | - | - | - |
| A2.5 | - | - | - |
| A3.1 | 1.4(1) | 0 | 0.2(0) |
| A3.2 | 5.7(4) | 5.5(3) | 21.5(108) |
| A11.1 | 0 | 5.5(3) | 0 |
| A11.2 | 5.7(4) | 31.4(17) | 8.7(44) |
| A11.3 | 0 | 3.7(2) | 0 |
| A23 | 4.3(3) | - | 3.9(20) |
| A24 | 2.9(2) | 27.7(15) | 15.3(77) |
| A24.2 | - | - | - |
| A24.3 | - | - | - |
| A25 | 1.4(1) | - | 6.9(35) |
| A26.1 | 4.3(3) | 9.2(5) | 5.9(30) |
| A26.2 | 7.2(5) | - | 1.0(5) |
| A26V | - | 3.7(2) | - |
| A28.1 | 10.1(7) | - | 1.6(8) |
| A28.2 | 1.4(1) | - | 7.5(38) |
| A29.1 | 1.4(1) | - | 1.4(7) |
| A29.2 | 10.1(7) | 1.8(1) | 5.3(27) |
| A30.1 | 8.6(6) | - | 4.9(25) |
| A30.2 | 1.4(1) | - | 0.2(1) |
| A30.3 | 7.2(5) | - | 3.9(20) |
| A31 | 4.3(3) | 7.4(4) | 6.9(35) |
| A32 | 2.8(2) | - | 7.1(36) |
| Aw33.1 | 8.6(6) | - | 2.5(13) |
| Aw33.2 | 2.8(2) | 16.6(9) | 1.2(6) |
| Aw34.1 | 1.4(1) | - | - |
| Aw34.2 | 14.5(10) | - | 0.8(4) |
| Aw36 | 5.9(4) | - | - |

| | | | |
|---|---|---|---|
| Table compiled from B. DuPont, Immunobiology of HLA, Vol. I, Histocompatibility Testing 1987, Springer-Verlag, New York 1989. N - negroid; A = Asian; C = caucasoid. Numbers in parenthesis represent the number of individuals included in the analysis. | | | |

The nomenclature used to describe peptide compounds follows the conventional practice wherein the amino group is presented to the left (the N-terminus) and the carboxyl group to the right (the C-terminus) of each amino acid residue. In the formulae, the amino-and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G.

The procedures used to identify peptides of the present invention generally follow the methods disclosed in Falk et al., Nature 351:290 (1991). Briefly, the methods involve large-scale isolation of MHC class I molecules, typically by immunoprecipitation or affinity chromatography, from the appropriate cell or cell line. Examples of other methods for isolation of the desired MHC molecule equally well known to the artisan include ion exchange chromatography, lectin chromatography, size exclusion, high performance ligand chromatography, and a combination of all of the above techniques.

In the typical case, immunoprecipitation is used to isolate the desired allele. A number of protocols can be used, depending upon the specificity of the antibodies used. For example, allele-specific mAb reagents can be used for the affinity purification of the HLA-A, HLA-B₁, and HLA-C molecules. Several mAb reagents for the isolation of HLA-A molecules are available. The monoclonal BB7.2 is suitable for isolating HLA-A2 molecules. Affinity columns prepared with these mAbs using standard techniques are successfully used to purify the respective HLA-A allele products.

In addition to allele-specific mAbs, broadly reactive anti-HLA-A, B, C mAbs, such as W6/32 and B9.12.1, and one anti-HLA-B, C mAb, B1.23.2, could be used in alternative affinity purification protocols as described in the example section below.

The peptides bound to the peptide binding groove of the isolated MHC molecules are eluted typically using acid treatment. Peptides can also be dissociated from class I molecules by a variety of standard denaturing means, such as heat, pH, detergents, salts, chaotropic agents, or a combination thereof.

Peptide fractions are further separated from the MHC molecules by reversed-phase high performance liquid chromatography (HPLC) and sequenced. Peptides can be separated by a variety of other standard means well known to the artisan, including filtration, ultrafiltration, electrophoresis, size chromatography, precipitation with specific antibodies, ion exchange chromatography, isoelectrofocusing, and the like.

Sequencing of the isolated peptides can be performed according to standard techniques such as Edman degradation (Hunkapiller, M.W., et al., Methods Enzymol. 91, 399 [1983]). Other methods suitable for sequencing include mass spectrometry sequencing of individual peptides as previously described (Hunt, et al., Science 225:1261 (1992) ). Amino acid sequencing of bulk heterogenous peptides (e.g., pooled HPLC fractions) from different class I molecules typically reveals a characteristic sequence motif for each class I allele.

Definition of motifs specific for different class I alleles allows the identification of potential peptide epitopes from an antigenic protein whose amino acid sequence is known. Typically, identification of potential peptide epitopes is initially carried out using a computer to scan the amino acid sequence of a desired antigen for the presence of motifs. The epitopic sequences are then synthesized. The capacity to bind MHC Class molecules is measured in a variety of different ways. One means is a Class I molecule binding assay as described in the related applications, noted above. Other alternatives described in the literature include inhibition of antigen presentation (Sette, et al., J. Immunol. 141:3893 (1991), in vitro assembly assays (Townsend, et al., Cell 62:285 (1990), and FACS based assays using mutated ells, such as RMA.S (Melief, et al., Eur. J. Immunol. 21:2963 (1991)).

Next, peptides that test positive in the MHC class I binding assay are assayed for the ability of the peptides to induce specific CTL responses in vitro. For instance, Antigen-presenting cells that have been incubated with a peptide can be assayed for the ability to induce CTL responses in responder cell populations. Antigen-presenting cells can be normal cells such as peripheral blood mononuclear cells or dendritic cells (Inaba, et al., J. Exp Med. 166:182 (1987); Boog, Eur. J. Immunol. 18:219 [1988]).

Alternatively, mutant mammalian cell lines that are deficient in their ability to load class I molecules with internally processed peptides, such as the mouse cell lines RMA-S (Kärre, et al.. Nature. 319:675 (1986); Ljunggren, et al., Eur. J. Immunol. 21:2963-2970 (1991)), and the human somatic T cell hybrid, T-2 (Cerundolo, et al., Nature 345:449-452 (1990)) and which have been transfected with the appropriate human class I genes are conveniently used, when peptide is added to them, to test for the capacity of the peptide to induce in vitro primary CTL responses. Other eukaryotic cell lines which could be used include various insect cell lines such as mosquito larvae (ATCC cell lines CCL 125, 126, 1660, 1591, 6585, 6586), silkworm (ATTC CRL 8851), armyworm (ATCC CRL 1711), moth (ATCC CCL 80) and Drosophila cell lines such as a Schneider cell line (see Schneider J. Embryol. Exp. Morphol. 27:353-365 [1927]).

Peripheral blood lymphocytes are conveniently isolated following simple venipuncture or leukapheresis of normal donors or patients and used as the responder cell sources of CTL precursors. In one embodiment, the appropriate antigen-presenting cells are incubated with 10-100 *µ*M of peptide in serum-free media for 4 hours under appropriate culture conditions. The peptide-loaded antigen-presenting cells are then incubated with the responder cell populations in vitro for 7 to 10 days under optimized culture conditions. Positive CTL activation can be determined by assaying the cultures for the presence of CTLs that kill radiolabeled target cells, both specific peptide-pulsed targets as well as target cells expressing endogenously processed form of the relevant virus or tumor antigen from which the peptide sequence was derived.

Specificity and MHC restriction of the CTL is determined by testing against different peptide target cells expressing appropriate or inappropriate human MHC class I. The peptides that test positive in the MHC binding assays and give rise to specific CTL responses are referred to herein as immunogenic peptides.

The immunogenic peptides can be prepared synthetically, or by recombinant DNA technology or from natural sources such as whole viruses or tumors. Although the peptide will preferably be substantially free of other naturally occurring host cell proteins and fragments thereof, in some embodiments the peptides can be synthetically conjugated to native fragments or particles.

The polypeptides or peptides can be a variety of lengths, either in their neutral (uncharged) forms or in forms which are salts, and either free of modifications such as glycosylation, side chain oxidation, or phosphorylation or containing these modifications, subject to the condition that the modification not destroy the biological activity of the polypeptides as herein described.

Desirably, the peptide will be as small as possible while still maintaining substantially all of the biological activity of the large peptide. When possible, it may be desirable to optimize peptides of the invention to a length of 9 or 10 amino acid residues, commensurate in size with endogenously processed viral peptides or tumor cell peptides that are bound to MHC class I molecules on the cell surface.

Peptides having the desired activity may be modified as necessary to provide certain desired attributes, e.g., improved pharmacological characteristics, while increasing or at least retaining substantially all of the biological activity of the unmodified peptide to bind the desired MHC molecule and activate the appropriate T cell. For instance, the peptides may be subject to various changes, such as substitutions, either conservative or non-conservative, where such changes might provide for certain advantages in their use, such as improved MHC binding. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gin; Ser, Thr; Lys, Arg; and Phe, Tyr. The effect of single amino acid substitutions may also be probed using D-amino acids. Such modifications may be made using well known peptide synthesis procedures, as described in e.g., Merrifield, Science 232:341-347 (1986), Barany and Merrifield, The Peptides. Gross and Meienhofer, eds. (N.Y., Academic Press), pp. 1-284 (1979); and Stewart and Young, Solid Phase Peptide Synthesis, (Rockford, III., Pierce), 2d Ed. (1984).

The peptides can also be modified by extending or decreasing the compound's amino acid sequence, e.g., by the addition or deletion of amino acids. The peptides or analogs can also be modified by altering the order or composition of certain residues, it being readily appreciated that certain amino acid residues essential for biological activity, e.g., those at critical contact sites or conserved residues, may generally not be altered without an adverse effect on biological activity. The non-critical amino acids need not be limited to those naturally occurring in proteins, such as L-α-amino acids, or their D-isomers, but may include non-natural amino acids as well, such as β-γ-δ-amino acids, as well as many derivatives of L-α-amino acids.

Typically, a series of peptides with single amino acid substitutions are employed to determine the effect of electrostatic charge, hydrophobicity, etc. on binding. For instance, a series of positively charged (e.g., Lys or Arg) or negatively charged (e.g., Glu) amino acid substitutions are made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding.

Amino acid substitutions are typically of single residues. Substitutions, deletions, insertions or any combination thereof may be combined to arrive at a final peptide. Substitutional variants are those in which at least one residue of a peptide has been removed and a different residue inserted in its place. Such substitutions generally are made in accordance with the following Table 2 when it is desired to finely modulate the characteristics of the peptide.

**TABLE 2**

| Original Residue | Exemplary Substitution |
|---|---|
| Ala | Ser |
| Arg | Lys, His |
| Asn | Gln |
| Asp | Glu |
| Cys | Ser |
| Gln | Asn |
| Glu | Asp |
| Gly | Pro |
| His | Lys; Arg |
| Ile | Leu; Val |
| Leu | Ile; Val |
| Lys | Arg; His |
| Met | Leu; Ile |
| Phe | Tyr; Trp |
| Ser | Thr |
| Thr | Ser |
| Trp | Tyr; Phe |
| Tyr | Trp; Phe |
| Val | Ile; Leu |

Substantial changes in function (e.g., affinity for MHC molecules or T cell receptors) are made by selecting substitutions that are less conservative than those in Table 2, i.e., selecting residues that differ more significantly in their effect on maintaining (a) the structure of the peptide backbone in the area of the substitution, for example as a sheet or helical conformation, (b) the charge or hydrophobicity of the molecule at the target site or (c) the bulk of the side chain. The substitutions which in general are expected to produce the greatest changes in peptide properties will be those in which (a) hydrophilic residue, e.g. seryl, is substituted for (or by) a hydrophobic residue, e.g. leucyl, isoleucyl, phenylalanyl, valyl or alanyl; (b) a residue having an electropositive side chain, e.g., lysl, arginyl, or histidyl, is substituted for (or by) an electronegative residue, e.g. glutamyl or aspartyl; or (c) a residue having a bulky side chain, e.g. phenylalanine, is substituted for (or by) one not having a side chain, e.g., glycine.

The peptides may also comprise isosteres of two or more residues in the immunogenic peptide. An isostere as defined here is a sequence of two or more residues that can be substituted for a second sequence because the steric conformation of the first sequence fits a binding site specific for the second sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks. See, generally, Spatola, Chemistry and Biochemistry of Amino Acids, peptides and Proteins, Vol. VII (Weinstein ed., 1983).

Modifications of peptides with various amino acid mimetics or unnatural amino acids are particularly useful in increasing the stability of the peptide in vivo. Stability can be assayed in a number of ways. For instance, peptidases and various biological media, such as human plasma and serum, have been used to test stability. See, e.g., Verhoef et al., Eur. J. Drug Metab. Pharmacokin. 11:291-302 (1986). Half life of the peptides of the present invention is conveniently determined using a 25% human serum (v/v) assay. The protocol is generally as follows. Pooled human serum (Type AB, non-heat inactivated) is delipidated by centrifugation before use. The serum is then diluted to 25% with RPMI tissue culture media and used to test peptide stability. At predetermined time intervals a small amount of reaction solution is removed and added to either 6 % aqueous trichloracetic acid or ethanol. The cloudy reaction sample is cooled (4°C) for 15 minutes and then spun to pellet the precipitated serum proteins. The presence of the peptides is then determined by reversed-phase HPLC using stability-specific chromatography conditions.

The peptides of the present invention or analogs thereof which have CTL stimulating activity may be modified to provide desired attributes other than improved serum half life. For instance, the ability of the peptides to induce CTL activity can be enhanced by linkage to a sequence which contains at least one epitope that is capable of inducing a T helper cell response. Particularly preferred immunogenic peptides/T helper conjugates are linked by a spacer molecule. The spacer is typically comprised of relatively small, neutral molecules, such as amino acids or amino acid mimetics, which are substantially uncharged under physiological conditions. The spacers are typically selected from, e.g., Ala, Gly, or other neutral spacers of nonpolar amino acids or neutral polar amino acids. It will be understood that the optionally present spacer need not be comprised of the same residues and thus may be a hetero- or homo-oligomer. When present, the spacer will usually be at least one or two residues, more usually three to six residues. Alternatively, the CTL peptide may be linked to the T helper peptide without a spacer.

The immunogenic peptide may be linked to the T helper peptide either directly or via a spacer either at the amino or carboxy terminus of the CTL peptide. The amino terminus of either the immunogenic peptide or the T helper peptide may be acylated. Exemplary T helper peptides include tetanus toxoid 830-843, influenza 307-319, malaria circumsporozoite 382-398 and 378-389.

In some embodiments it may be desirable to include in the pharmaceutical compositions of the invention at least one component which primes CTL. Lipids have been identified as agents capable of priming CTL in vivo against viral antigens. For example, palmitic acid residues can be attached to the alpha and epsilon amino groups of a Lys residue and then linked, e.g., via one or more linking residues such as Gly, Gly-Gly-, Ser, Ser-Ser, or the like, to an immunogenic peptide. The lipidated peptide can then be injected directly in a micellar form, incorporated into a liposome or emulsified in an adjuvant, e.g., incomplete Freund's adjuvant. In a preferred embodiment a particularly effective immunogen comprises palmitic acid attached to alpha and epsilon amino groups of Lys, which is attached via linkage, e.g., Ser-Ser, to the amino terminus of the immunogenic peptide.

As another example of lipid priming of CTL responses, E. coli lipoproteins, such as tripalmitoyl-S-glycerylcysteinlyseryl-serine (P₃CSS) can be used to prime virus specific CTL when covalently attached to an appropriate peptide. See, Deres et al., Nature 342:561-564 (1989). Peptides of the invention can be coupled to P₃CSS, for example, and the lipopeptide administered to an individual to specifically prime a CTL response to the target antigen. Further, as the induction of neutralizing antibodies can also be primed with P₃CSS conjugated to a peptide which displays an appropriate epitope, the two compositions can be combined to more effectively elicit both humoral and cell-mediated responses to infection.

In addition, additional amino acids can be added to the termini of a peptide to provide for ease of linking peptides one to another, for coupling to a carrier support, or larger peptide, for modifying the physical or chemical properties of the peptide or oligopeptide, or the like. Amino acids such as tyrosine, cysteine, lysine, glutamic or aspartic acid, or the like, can be introduced at the C- or N-terminus of the peptide or oligopeptide. Modification at the C terminus in some cases may alter binding characteristics of the peptide. In addition, the peptide or oligopeptide sequences can differ from the natural sequence by being modified by terminal-NH₂ acylation, e.g., by alkanoyl (C₁-C₂₀) or thioglycolyl acetylation, terminal-carboxyl amidation, e.g., ammonia, methylamine, etc. In some instances these modifications may provide sites for linking to a support or other molecule.

The peptides can be prepared in a wide variety of ways. Because of their relatively short size, the peptides can be synthesized in solution or on a solid support in accordance with conventional techniques. Various automatic synthesizers are commercially available and can be used in accordance with known protocols. See, for example, Stewart and Young, Solid Phase Peptides Synthesis. 2d. ed., Pierce Chemical Co. (1984), supra.

Alternatively, recombinant DNA technology may be employed wherein a nucleotide sequence which encodes an immunogenic peptide of interest is inserted into an expression vector, transformed or transfected into an appropriate host cell and cultivated under conditions suitable for expression. These procedures are generally known in the art, as described generally in Sambrook et al., Molecular Cloning, A laboratory Mannal, Cold Spring Harbor Press, Cold Spring Harbor, New York (1982). Thus, fusion proteins which comprise one or more peptide sequences of the can be used to present the appropriate T cell epitope.

As the coding sequence for peptides of the length contemplated herein can be synthesized by chemical techniques, for example, the phosphotriester method of Matteucci et al., J. Am. Chem. Soc. 103:3185 (1981), modification can be made simply by substituting the appropriate base(s) for those encoding the native peptide sequence. The coding sequence can then be provided with appropriate linkers and ligated into expression vectors commonly available in the art, and the vectors used to transform suitable hosts to produce the desired fusion protein. A number of such vectors and suitable host systems are now available. For expression of the fusion proteins, the coding sequence will be provided with operably linked start and stop codons, promoter and terminator regions and usually a replication system to provide an expression vector for expression in the desired cellular host. For example, promoter sequences compatible with bacterial hosts are provided in plasmids containing convenient restriction sites for insertion of the desired coding sequence. The resulting expression vectors are transformed into suitable bacterial hosts. Of course, yeast or mammalian cell hosts may also be used, employing suitable vectors and control sequences.

The peptides of the present invention and pharmaceutical and vaccine compositions thereof are useful for administration to mammals, particularly humans, to treat and/or prevent hepatitis C infection.

For pharmaceutical compositions, the immunogenic peptides of the invention are administered to an individual already suffering from cancer or infected with the virus of interest. Those in the incubation phase or the acute phase of infection can be treated with the immunogenic peptides separately or in conjunction with other treatments, as appropriate. In therapeutic applications, compositions are administered to a patient in an amount sufficient to elicit an effective CTL response to the virus or tumor antigen and to cure or at least partially arrest symptoms and/or complications. An amount adequate to accomplish this is defined as "therapeutically effective dose.' Amounts effective for this use will depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 *µ*g to about 5000 *µ*g of peptide for a 70 kg patient, followed by boosting dosages of from about 1.0 *µ*g to about 1000 *µ*g of peptide pursuant to a boosting regimen over weeks to months depending upon the patient's response and condition by measuring specific CTL activity in the patient's blood. It must be kept in mind that the peptides and compositions of the present invention may generally be employed in serious disease states, that is, life-threatening or potentially life threatening situations. In such cases, in view of the minimization of extraneous substances and the relative nontoxic nature of the peptides, it is possible and may be felt desirable by the treating physician to administer substantial excesses of these peptide compositions.

For therapeutic use, administration should begin at the first sign of viral infection or the detection or surgical removal of tumors or shortly after diagnosis in the case of acute infection. This is followed by boosting doses until at least symptoms are substantially abated and for a period thereafter. In chronic infection, loading doses followed by boosting doses may be required.

Treatment of an infected individual with the compositions of the invention may hasten resolution of the infection in acutely infected individuals. For those individuals susceptible (or predisposed) to developing chronic infection the compositions are particularly useful in methods for preventing the evolution from acute to chronic infection. Where the susceptible individuals are identified prior to or during infection, for instance, as described herein, the composition can be targeted to them, minimizing need for administration to a larger population.

The peptide compositions can also be used for the treatment of chronic infection and to stimulate the immune system to eliminate virus-infected cells in carriers. It is important to provide an amount of immuno-potentiating peptide in a formulation and mode of administration sufficient to effectively stimulate a cytotoxic T cell response. Thus, for treatment of chronic infection, a representative dose is in the range of about 1.0 *µ*g to about 5000 *µ*g, preferably about 5 *µ*g to 1000 *µ*g for a 70 kg patient per dose. Immunizing doses followed by boosting doses at established intervals, e.g., from one to four weeks, may be required, possibly for a prolonged period of time to effectively immunize an individual. In the case of chronic infection, administration should continue until at least clinical symptoms or laboratory tests indicate that the viral infection has been eliminated or substantially abated and for a period thereafter.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the immunogenic peptides dissolved or suspended in an acceptable carrier, preferably an aqueous carrier. A variety of aqueous carriers may be used, e.g., water, buffered water, 0.8% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of CTL stimulatory peptides of the invention in the pharmaceutical formulations can vary widely, i.e., from less than about 0.1 %, usually at or at least about 2% to as much as 20% to 50% or more by weight, and will be selected primarily by fluid volumes, viscosities, etc., in accordance with the particular mode of administration selected.

The peptides of the invention may also be administered via liposomes, which serve to target the peptides to a particular tissue, such as lymphoid tissue, or targeted selectively to infected cells, as well as increase the half-life of the peptide composition. Liposomes include emulsions, foams, micelles, insoluble monolayers, liquid crystals, phospholipid dispersions, lamellar layers and the like. In these preparations the peptide to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes either filled or decorated with a desired peptide of the invention can be directed to the site of lymphoid cells, where the liposomes then deliver the selected therapeutic/immunogenic peptide compositions. Liposomes for use in the invention are formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9:467 (1980), U.S. Patent Nos. 4,235,871, 4,501,728, 4,837,028, and 5,019,369, incorporated herein by reference.

For targeting to the immune cells, a ligand to be incorporated into the liposome can include, e.g., antibodies or fragments thereof specific for cell surface determinants of the desired immune system cells. A liposome suspension containing a peptide may be administered intravenously, locally, topically, etc. in a dose which varies according to, inter alia, the manner of administration, the peptide being delivered, and the stage of the disease being treated.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95 % of active ingredient, that is, one or more peptides of the invention, and more preferably at a concentration of 25%-75%.

For aerosol administration, the immunogenic peptides are preferably supplied in finely divided form along with a surfactant and propellant. Typical percentages of peptides are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included, as desired, as with, e.g., lecithin for intranasal delivery.

In another aspect the present invention is directed to vaccines which contain as an active ingredient an immunogenically effective amount of an immunogenic peptide as described herein or a nucleic acid encoding it. The peptide(s) or nucleic acids may be introduced into a host, including humans, linked to its own carrier or as a homopolymer or heteropolymer of active peptide units. Such a polymer has the advantage of increased immunological reaction and, where different peptides are used to make up the polymer, the additional ability to induce antibodies and/or CTLs that react with different antigenic determinants of the virus or tumor cells. Useful carriers are well known in the art, and include, e.g., thyroglobulin, albumins such as human serum albumin, tetanus toxoid, polyamino acids such as poly(lysine:glutamic acid), influenza, hepatitis B virus core protein, hepatitis B virus recombinant vaccine and the like. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art. And, as mentioned above, CTL responses can be primed by conjugating peptides of the invention to lipids, such as P₃CSS. Upon immunization with a peptide composition as described herein, via injection, aerosol, oral, transdermal or other route, the immune system of the host responds to the vaccine by producing large amounts of CTLs specific for the desired antigen, and the host becomes at least partially immune to later infection, or resistant to developing chronic infection.

Vaccine compositions containing the peptides of the invention are administered to a patient susceptible to or otherwise at risk of viral infection or cancer to elicit an immune response against the antigen and thus enhance the patient's own immune response capabilities. Such an amount is defined to be an "immunogenically effective dose." In this use, the precise amounts again depend on the patient's state of health and weight, the mode of administration, the nature of the formulation, etc., but generally range from about 1.0 *µ*g to about 5000 *µ*g per 70 kilogram patient, more commonly from about 10 *µ*g to about 500 *µ*g mg per 70 kg of body weight.

In some instances it may be desirable to combine the peptide vaccines of the invention with vaccines which induce neutralizing antibody responses to the virus of interest, particularly to viral envelope antigens.

For therapeutic or immunization purposes, the peptides of the invention can also be expressed by attenuated viral hosts, such as vaccinia or fowlpox. This approach involves the use of vaccinia virus as a vector to express nucleotide sequences that encode the peptides of the invention. Upon introduction into an acutely or chronically infected host or into a non-infected host, the recombinant vaccinia virus expresses the immunogenic peptide, and thereby elicits a host CTL response. Vaccinia vectors and methods useful in immunization protocols are described in, e.g., U.S. Patent No. 4,722,848, incorporated herein by reference. Another vector is BCG (Bacille Calmette Guerin). BCG vectors are described in Stover et al. (Nature 351:456-460 (1991)) which is incorporated herein by reference. A wide variety of other vectors useful for therapeutic administration or immunization of the peptides of the invention, e.g., Salmonella typhi vectors and the like, will be apparent to those skilled in the art from the description herein.

Nucleic acids encoding one or more of the peptides of the invention can also be admisitered to the patient. This approach is described, for instance, in Wolff et. al., Science 247: 1465-1468 (1990) as well as U.S. Patent Nos. 5,580,859 and 5,589,466.

A preferred means of administering nucleic acids encoding the peptides of the invention uses minigene constructs encoding multiple epitopes of the invention. To create a DNA sequence encoding the selected CTL epitopes (minigene) for expression in human cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte epitopes, a leader (signal) sequence, and an endoplasmic reticulum retention signal. In addition, MHC presentation of CTL epitopes may be improved by including synthetic (e.g. poly-alanine) or naturally-occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. he ends of the oligonucleotides are joined using T4 DNA ligase. This synthetic minigene, encoding the CTL epitope polypeptide, can then cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an *E. coli* origin of replication; and an *E. coli* selectable marker (e.g. ampicillin or kanamycin resistance). Numerous promoters can be used for this purpose, e.g., the human cytomegalovirus (hCMV) promoter. *See,* U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immunostimulatory sequences (ISSs or CpGs) play a role in the immunogenicity of DNA vaccines. These sequences could be included in the vector, outside the minigene coding sequence, if found to enhance immunogenicity.

In some embodiments, a bicistronic expression vector, to allow production of the minigene-encoded epitopes and a second protein included to enhance or decrease immunogenicity can be used. Examples of proteins or polypeptides that could beneficially enhance the immune response if co-expressed include cytokines (e.g., IL2, IL12, GM-CSF), cytokine-inducing molecules (e.g. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (e.g. TGF-β) may be beneficial in certain diseases.

Once an expression vector is selected, the minigene is cloned into the polylinker region downstream of the promoter. This plasmid is transformed into an appropriate *E. coli* strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Therapeutic quantities of plasmid DNA are produced by fermentation in *E. coli,* followed by purification. Aliquots from the working cell bank are used to inoculate fermentation medium (such as Terrific Broth), and grown to saturation in shaker flasks or a bioreactor according to well known techniques. Plasmid DNA can be purified using standard bioseparation technologies such as solid phase anion-exchange resins supplied by Quiagen. If required, supercoiled DNA can be isolated from the open circular and linear forms using gel electrophoresis or other methods.

Purified plasmid DNA can be prepared for injection using a variety of formulations. The simplest of these is reconstitution of lyophilized DNA in sterile phosphate-buffer saline (PBS). This approach, known as "naked DNA," is currently being used for intramuscular (IM) administration in clinical trials. To maximize the immunotherapeutic effects of minigene DNA vaccines, an alternative method for formulating purified plasmid DNA may be desirable. A variety of methods have been described, and new techniques may become available. Cationic lipids can also be used in the formulation (*see, e.g.,* as described by Debs and Zhu (1993) WO 93/24640; Mannino and Gould-Fogerite (1988) BioTechniques 6(7): 682-691; Rose U.S. Pat No. 5,279,833; Brigham (1991) WO 91/06309; and Feigner et al. (1987) Proc. Natl. Acad. Sci. USA 84: 7413-7414). In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

The nucleic acids can also be administered using ballistic delivery as described, for instance, in U.S. Patent No. 5,204,253. Particles comprised solely of DNA can be administered. Alternatively, DNA can be adhered to particles, such as gold particles.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used will be dependent on the final formulation. Electroporation can be used for "naked" DNA, whereas cationic lipids allow direct *in vitro* transfection. A plasmid expressing green fluorescent protein (GFP) can be co-transfected to allow enrichment of transfected cells using fluorescence activated cell sorting (FACS). These cells are then chromium-51 labeled and used as target cells for epitope-specific CTL lines. Cytolysis, detected by 51Cr release, indicates production of MHC presentation of minigene-encoded CTL epitopes.

*In vivo* immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for *in vivo* induction of CTLs.

Antigenic peptides may be used to elicit CTL ex vivo, as well. The resulting CTL, can be used to treat chronic infections (viral or bacterial) or tumors in patients that do not respond to other conventional forms of therapy, or will not respond to a peptide vaccine approach of therapy. Ex vivo CTL responses to a particular pathogen (infectious agent or tumor antigen) are induced by incubating in tissue culture the patient's CTL precursor cells (CTLp) together with a source of antigen-presenting cells (APC) and the appropriate immunogenic peptide. After an appropriate incubation time (typically 1-4 weeks), in which the CTLp are activated and mature and expand into effector CTL, the cells are infused back into the patient, where they will destroy their specific target cell (an infected cell or a tumor cell).

The peptides may also find use as diagnostic reagents. For example, a peptide of the invention may be used to determine the susceptibility of a particular individual to a treatment regimen which employs the peptide or related peptides, and thus may be helpful in modifying an existing treatment protocol or in determining a prognosis for an affected individual. In addition, the peptides may also be used to predict which individuals will be at substantial risk for developing chronic infection.

The following example is offered by way of illustration, not by way of limitation.

### Example 1

Class I antigen isolation was carried out as described in the related applications, noted above. Naturally processed peptides were then isolated and sequenced as described there. An allele-specific motif and algorithms were determined and quantitative binding assays were carried out.

Using the motifs identified above for HLA-A2.1 allele amino acid sequences from various antigenic proteins were analyzed for the presence of these motifs. Table 3 provides the results of these searches. Binding affinities are expressed as percentage of binding compared to standard peptide in the assays as described in the related applications are presented.

**Table 3**

| SEQ ID NO: | AA | SEQUENCE | SOURCE |
|---|---|---|---|
| 1 | 9 | ILSPGALVV | HCV NS4 1891 |
| 2 | 9 | LLFLLLADA | HCV NS1/E2 726 |
| 3 | 9 | WMNRLIAFA | HCV NS4 1920 |
| 4 | 9 | VLVGGVLAA | HCV NS4 1666 |
| 5 | 9 | LLQLTVWGI | HIV-1 ENV 61 |
| 6 | 9 | MTNNPPIPV | HIV-1 GAG 34 |
| 7 | 9 | FLQSRPEPT | HIV-1 GAG 45 |
| 8 | 9 | LTFGWCFKL | HIV-1 NEF 62 |
| 9 | 9 | TLNFPISPI | HIV-1 POL 96 |
| 10 | 9 | YTAFTIPSI | HIV-1 POL 83 |
| 11 | 9 | TLWQRPLVT | HIV-1 POL 65 |
| 12 | 9 | KAACWWAGI | HIV-1 POL 65 |
| 13 | 9 | ALVEICTEM | HIV-1 POL 52 |
| 14 | 9 | LVGPTPVNI | HIV-1 POL 100 |
| 15 | 9 | RAMASDFNL | HIV-1 POL 78 |
| 16 | 9 | QLLFIHFRI | HIV-1 VPR 80 |
| 17 | 9 | AIIRILQQL | HIV-1 VPR 76 |
| 18 | 9 | RILQQLLFI | HIV-1 VPR 72 |
| 19 | 9 | KLVGKLNWA | HIV-1 POL 87 |
| 20 | 10 | WMTNNPPIPV | HIV-1 GAG 34 |
| 21 | 10 | PLTFGWCFKL | HIV-1 NEF 62 |
| 22 | 10 | LTFGWCFKLV | HIV-1 NEF 62 |
| 23 | 10 | MASDFNLPPV | HIV-1 POL 70 |
| 24 | 10 | CTLNFPISPI | HIV-1 POL 96 |
| 25 | 10 | KLNWASQIYA | HIV-1 POL 61 |
| 26 | 10 | KMIGGIGGFI | HIV-1 POL 96 |
| 27 | 10 | FLPS(X)YFPSV X=K-BIOTIN | Biotinylated HBc 1 |
| 27 | 10 | FLPS(X)YFPSV X=K-BIOTIN | Biotinylated HBc 1 |
| 27 | 10 | FLPS(X)YFPSV X=K-BIOTIN | Biotinylated HBc 1 |
| 28 | 10 | FLPSD(X)FPSV X=K-BIOTIN | Biotinylated HBc 1 |
| 28 | 10 | FLPSD(X)FPSV X=K-BIOTIN | Biotinylated HBc 1 |
| 29 | 9 | YMDDVVLGV | HBV pol 538-546 s |
| 30 | 10 | FLPSZFFPSV | HBc 18-27 SAAS @p5 |
| 30 | 10 | FLPSZFFPSV | HBc 18-27 SAAS @p5 |
| 31 | 10 | FLPSDAFPSV | HBc 18-27 alanine |
| 32 | 10 | FLPSAFFPSV | HBc 18-27 alanine |
| 33 | 10 | FLPSDFYPSV | HBc 18-27 alanine |
| 34 | 10 | FLPSDFFASV | HBc 18-27 alanine |
| 35 | 10 | FLPSDFFPAV | HBc 18-27 alanine |
| 36 | 10 | FLASDFFPSV | HBc 18-27 alanine |
| 37 | 10 | ALPSDFFPSV | HBc 18-27 alanine |
| 38 | 10 | YLPSDFFPSV | HBc 18-27 SAAS |
| 39 | 10 | FMPSDFFPSV | HBc 18-27 SAAS |
| 40 | 10 | FLKSDFFPSV | HBc 18-27 SAAS |
| 41 | 10 | FLPSEFFPSV | HBc 18-27 SAAS |
| 42 | 10 | FLPSDFYPSV | HBc 18-27 SAAS |
| 43 | 10 | FLPSDFFKSV | HBc 18-27 SAAS |
| 44 | 10 | FLPSDFFPKV | HBc 18-27 SAAS |

## Claims

1. An isolated peptide of less than 15 amino acids, which comprises the amino acid sequence LLFLLLADA (SEQ ID NO:2).

2. The isolated peptide of claim 1, wherein the peptide consists of the amino acid sequence LLFLLLADA (SEQ ID NO:2).

3. The isolated peptide of claim 1 or 2, wherein the peptide is linked to another peptide.

4. The isolated peptide of claim 1 or 2, wherein the peptide is linked to a second molecule.

5. The isolated peptide of claim 4, wherein the second molecule is a lipid, a T helper epitope, a cytotoxic T lymphocyte (CTL) epitope, or a carrier molecule.

6. A composition which comprises an isolated peptide of any one of claims 1 to 5 complexed with an HLA A2.1 molecule present on an antigen-presenting cell.

7. A pharmaceutical composition which comprises a peptide of any one of claims 1 to 5 and a pharmaceutically acceptable excipient.

8. An isolated nucleic acid for use in the treatment or prevention of Hepatitis C virus (HCV) infection, which nucleic acid comprises a nucleotide sequence encoding a polypeptide that comprises CTL epitopes from HCV joined to one another, wherein one or more epitopes comprise the amino acid sequence LLFLLLADA.

9. An isolated nucleic acid which comprises a nucleotide sequence encoding a polypeptide that comprises CTL epitopes from HCV joined to one another, wherein one or more epitopes comprise the amino acid sequence LLFLLLADA, and wherein one or more further epitopes consists of the amino acid sequence: ILSPGALW (SEQ ID NO:1); LLFLLLADA (SEQ ID NO:2); WMNRLIAFA (SEQ ID NO:3); or VLVGGVLAA (SEQ ID NO:4).

10. The isolated nucleic acid of claim 9, wherein the polypeptide comprises further epitopes consisting of the amino acid sequences ILSPGALVV (SEQ ID NO:1), LLFLLLADA (SEQ ID NO:2), WMNRLIAFA (SEQ ID NO:3), and VLVGGVLAA (SEQ ID NO:4).

11. The isolated nucleic acid of claim 9, wherein the further epitopes consist of the amino acid sequences: YMDDVVLGV (SEQ ID NO:29), FLPSZFFPSV (SEQ ID NO:30), FLPSDAFPSV (SEQ ID NO:31), FLPSAFFPSV (SEQ ID NO:32), FLPSDFAPSV (SEQ ID NO:33), FLPSDFFASV (SEQ ID NO:34), FLPSDFFPAV (SEQ ID NO:35), FLASDFFPSV (SEQ ID NO:36), ALPSDFFPSV (SEQ ID NO:37), YLPSDFFPSV (SEQ ID NO:38), FMPSDFFPSV (SEQ ID NO:39), FLKSDFFPSV (SEQ_ ID NO:40), FLESEFFPSV (SEQ ID NO:41), FLPSDFYPSV (SEQ ID NO:42), FLPSDFFKSV (SEQ ID NO:43), and FLPSDFFPKV (SEQ ID NO:44).

12. The isolated nucleic acid of any one of claims 9 to 11, wherein the polypeptide comprises flanking sequences adjacent to one or more of the CTL epitopes.

13. A polypeptide encoded by the nucleic acid of any one of claims 9 to 12.

14. A pharmaceutical composition comprising an isolated nucleic acid of any one of claims 9 to 12 and a pharmaceutically acceptable excipient.

15. An ex vivo method for using an immunogenic peptide composition, which comprises providing an isolated peptide of less than 15 amino acids that comprises the amino acid sequence: LLFLLLADA (SEQ ID NO:2); complexing the immunogenic peptide with an HLA A2.1 molecule; and contacting in vitro an HLA A2.1-restricted CTL with the complex of the peptide and the HLA A2.1 molecule, whereby a CTL response is induced.

16. The method of claim 15, wherein the peptide consists of the amino acid sequence: LLFLLLADA (SEQ ID NO:2).

17. The use of an immunogenic peptide for the manufacture of a medicament for the treatment or prevention of HCV infection, wherein the peptide is less than 15 amino acids and comprises the amino sequence LLFLLLADA (SEQ ID NO:2).

18. The use of claim 17, wherein the peptide consists of the amino acid sequence LLFLLLADA (SEQ ID NO:2).

19. The use of a nucleic acid for the manufacture of a medicament for the treatment or prevention of HCV infection, wherein the nucleic acid comprises a nucleotide sequence encoding a polypeptide that comprises CTL epitopes from HCV joined to one another, wherein one or more epitopes comprise the amino acid sequence LLFLLLADA (SEQ ID NO:2).

20. The use of claim 19, wherein one of the epitopes consists of the amino acid sequence LLFLLLADA (SEQ ID NO:2).

21. The use of claim 19, wherein the polypeptide comprises further epitopes consisting of the amino acid sequences: ILSPGALVV (SEQ ID NO:1), LLFLLLADA (SEQ ID NO:2), WMNRLIAFA (SEQ ID NO:3), and VLVGGVLAA (SEQ ID NO:4).

22. The use of any one of claims 19 to 21, wherein the polypeptide comprises flanking sequences adjacent to one or more of the CTL epitopes.

23. The method of claim 15 or 16 wherein the HLA A2.1 molecule is expressed on a cell.

## Patentansprüche

1. Isoliertes Peptid von weniger als 15 Aminosäuren, das die Aminosäuresequenz LLFLLLADA umfasst (SEQ ID NO: 2) umfasst.

2. Isoliertes Peptid nach Anspruch 1, wobei das Peptid aus der Aminosäuresequenz LLFLLLADA (SEQ ID NO: 2) besteht.

3. Isoliertes Peptid nach Anspruch 1 oder 2, wobei das Peptid mit einem weiteren Peptid verknüpft ist.

4. Isoliertes Peptid nach Anspruch 1 oder 2, wobei das Peptid mit einem zweiten Molekül verknüpft ist.

5. Isoliertes Peptid nach Anspruch 4, wobei das zweite Molekül ein Lipid, ein T-Helfer-Epitop, ein zytotoxisches T-Lymphozyten (CTL) -Epitop oder ein Trägermolekül ist.

6. Zusammensetzung, die ein isoliertes Peptid nach einem der Ansprüche 1 bis 5 umfasst, das mit einem auf einer Antigen-präsentierenden Zelle vorhandenen HLA A2.1-Molekül komplexiert ist.

7. Pharmazeutische Zusammensetzung, die ein Peptid nach einem der Ansprüche 1 bis 5 und einen pharmazeutisch verträglichen Trägerstoff umfasst.

8. Isolierte Nukleinsäure zur Verwendung bei der Behandlung oder Prävention einer Hepatitis-C-Virus (HCV) -Infektion, wobei die Nukleinsäure eine Nukleotidsequenz umfasst, die für ein Polypeptid kodiert, das miteinander verbundene CTL-Epitope von HCV umfasst, wobei ein oder mehrere Epitope die Aminosäuresequenz LLFLLLADA umfassen.

9. Isolierte Nukleinsäure, die eine Nukleotidsequenz umfasst, die für ein Polypeptid kodiert, das miteinander verbundene CTL-Epitope von HCV umfasst, wobei ein oder mehrere Epitope die Aminosäuresequenz LLFLLLADA umfassen und wobei ein oder mehrere weitere Epitope aus der Aminosäuresequenz ILSPGALVV (SEQ ID NO: 1); LLFLLLADA (SEQ ID NO: 2); WMNRLIAFA (SEQ ID NO: 3) oder VLVGGVLAA (SEQ ID NO: 4) bestehen.

10. Isolierte Nukleinsäure nach Anspruch 9, wobei das Polypeptid weitere Epitope umfasst, die aus den Aminosäuresequenzen ILSPGALVV (SEQ ID NO: 1), LLFLLLADA (SEQ ID NO: 2), WMNRLIAFA (SEQ ID NO: 3) und VLVGGVLAA (SEQ ID NO: 4) bestehen.

11. Isolierte Nukleinsäure nach Anspruch 9, wobei die weiteren Epitope aus den Aminosäuresequenzen YMDDVVLGV (SEQ ID NO: 29), FLPSZFFPSV (SEQ ID NO: 30), FLPSDAFPSV (SEQ ID NO:31), FLPSAFFPSV (SEQ ID NO:32), FLPSDFAPSV (SEQ ID NO:33), FLPSDFFASV (SEQ ID NO:34), FLPSDFFPAV (SEQID NO:35), FLASDFFPSV (SEQ ID NO:36), ALPSDFFPSV (SEQ ID NO:37), YLPSDFFPSV (SEQ ID NO:38), FMPSDFFPSV (SEQ ID NO:39), FLKSDFFPSV (SEQ ID NO:40), FLPSEFFPSV (SEQ ID NO:41), FLPSDFYPSV (SEQ ID NO:42), FLPSDFFKSV (SEQ ID NO:43) und FLPSDFFPKV (SEQ ID NO:44) bestehen.

12. Isolierte Nukleinsäure nach einem der Ansprüche 9 bis 11, wobei das Polypeptid flankierende Sequenzen benachbart zu einem oder mehreren der CTL-Epitope umfasst.

13. Polypeptid, das durch die Nukleinsäure nach einem der Ansprüche 9 bis 12 kodiert wird.

14. Pharmazeutische Zusammensetzung, umfassend eine isolierte Nukleinsäure nach einem der Ansprüche 9 bis 12 und einen pharmazeutisch verträglichen Trägerstoff.

15. Ex vivo Verfahren zur Verwendung einer immunogenen Peptidzusammensetzung, welches das Bereitstellen eines isolierten Peptids von weniger als 15 Aminosäuren umfasst, das die Aminosäuresequenz LLFLLLADA (SEQ ID NO: 2) umfasst, Komplexieren des immunogenen Peptids mit einem HLA A2.1-Molekül und in vitro Kontaktieren eines HLA A2.1-beschränkten CTL mit dem Komplex aus dem Peptid und dem HLA A2.1-Molekül, wodurch eine CTL-Antwort induziert wird.

16. Verfahren nach Anspruch 15, wobei das Peptid aus der Aminosäuresequenz: LLFLLLADA (SEQ ID NO: 2) besteht.

17. Verwendung eines immunogenen Peptids für die Herstellung eines Medikaments zur Behandlung oder Prävention einer HCV-Infektion, wobei das Peptid weniger als 15 Aminosäuren aufweist und die Aminosäuresequenz LLFLLLADA (SEQ ID NO: 2) umfasst.

18. Verwendung nach Anspruch 17, wobei das Peptid aus der Aminosäuresequenz LLFLLLADA (SEQ ID NO: 2) besteht.

19. Verwendung einer Nukleinsäure zur Herstellung eines Medikaments zur Behandlung oder Prävention einer HCV-Infektion, wobei die Nukleinsäure eine Nukleotidsequenz umfasst, die für ein Polypeptid kodiert, das miteinander verbundene CTL-Epitope von HCV umfasst, wobei ein oder mehrere Epitope die Aminosäuresequenz LLFLLLADA (SEQ ID NO: 2) umfassen.

20. Verwendung nach Anspruch 19, wobei eines der Epitope aus der Aminosäuresequenz LLFLLLADA (SEQ ID NO: 2) besteht.

21. Verwendung nach Anspruch 19, wobei das Polypeptid weitere Epitope umfasst, die aus den Aminosäuresequenzen ILSPGALVV (SEQ ID NO: 1), LLFLLLADA (SEQ ID NO: 2), WMNRLIAFA (SEQ ID NO: 3) und VLVGGVLAA (SEQ ID NO: 4) bestehen.

22. Verwendung nach einem der Ansprüche 19 bis 21, wobei das Polypeptid flankierende Sequenzen benachbart zu einem oder mehreren der CTL-Epitope umfasst.

23. Verfahren nach den Ansprüchen 15 oder 16, wobei das HLA A2.1-Molekül auf einer Zelle exprimiert wird.

## Revendications

1. Peptide isolé de moins de 15 acides aminés, qui comprend la séquence d'acides aminés LLFLLLADA (SEQ ID NO : 2).

2. Peptide isolé selon la revendication 1, dans lequel le peptide consiste en la séquence d'acides aminés LLFLLLADA (SEQ ID NO : 2).

3. Peptide isolé selon la revendication 1 ou 2, dans lequel le peptide est lié à un autre peptide.

4. Peptide isolé selon la revendication 1 ou 2, dans lequel le peptide est lié à une seconde molécule.

5. Peptide isolé selon la revendication 4, dans lequel la seconde molécule est un lipide, un épitope auxiliaire de cellule T, un épitope de lymphocyte T cytotoxique (CTL), ou une molécule en charge.

6. Composition qui comprend un peptide isolé selon l'une quelconque des revendications de 1 à 5, complexé avec une molécule A2.1 HLA présente sur une cellule présentant un antigène.

7. Composition pharmaceutique qui comprend un peptide selon l'une quelconque des revendications 1 à 5 et un excipent pharmaceutiquement acceptable.

8. Acide nucléique isolé pour une utilisation dans le traitement ou la prévention d'une infection par le virus de l'hépatite C (VHC), lequel acide nucléique comprend une séquence de nucléotides codant pour un polypeptide qui comprend des épitopes de CTL provenant du VHC joints les uns aux autres, dans lequel un ou plusieurs épitopes comprend (comprennent) la séquence d'acides aminés LLFLLLADA.

9. Aide nucléique isolé qui comprend une séquence de nucléotides codant pour un polypeptide qui comprend des épitopes de CTL provenant du VHC joints les uns aux autres, dans lequel un ou plusieurs épitopes comprend (comprennent) la séquence d'acides aminés LLFLLLADA, et dans lequel un ou plusieurs autres épitopes consiste (consistent) en la séquence d'acides aminés : ILSPGALVV (SEQ ID NO : 1) ; LLFLLLADA (SEQ ID NO : 2) ; WMNRLIAFA (SEQ ID NO : 3) ; ou VLVGGVLAA (SEQ ID NO : 4).

10. Acide nucléique isolé selon la revendication 9, dans lequel le polypeptide comprend d'autres épitopes consistant en les séquences d'acides aminés ILSPGALVV (SEQ ID NO : 1), LLFLLLADA (SEQ ID NO : 2), WMNRLIAFA (SEQ ID NO : 3), et VLVGGVLAA (SEQ ID NO : 4).

11. Acide nucléique isolé selon la revendication 9, dans lequel les autres épitopes consistent en les séquences d'acides aminés : YMDDVVLGV (SEQ ID NO : 29), FLPSZFFPSV (SEQ ID NO : 30), FLPSDAFPSV (SEQ ID NO : 31), FLPSAFFPSV (SEQID NO : 32), FLPSDFAPSV (SEQ ID NO : 33), FLPSDFFASV (SEQ ID NO : 34), FLPSDFFPAV (SEQ ID NO : 35), FLASDFFPSV (SEQ ID NO : 36), ALPSDFFPSV (SEQ ID NO : 37), YLPSDFFPSV (SEQ ID NO : 38), FMPSDFFPSV(SEQ ID NO : 39), FLKSDFFPSV (SEQ ID NO : 40), FLPSEFFPSV (SEQ ID NO : 41), FLPSDFYPSV (SEQ ID NO : 42), FLPSDFFKSV (SEQ ID NO : 43), et FLPSDFFPKV (SEQ ID NO : 44) .

12. Acide nucléique isolé selon l'une quelconque des revendications 9 à 11, dans lequel le polypeptide comprend des séquences flanquantes, adjacentes à un ou plusieurs des épitopes de CTL.

13. Polypeptide codé par l'acide nucléique selon l'une quelconque des revendications 9 à 12.

14. Composition pharmaceutique comprenant un acide nucléique isolé selon l'une quelconque des revendications 9 à 12 et un excipient pharmaceutiquement acceptable.

15. Méthode ex vivo destinée à utiliser une composition de peptide immunogène, qui comprend la fourniture d'un peptide isolé de moins de 15 acides aminés qui comprend la séquence d'acides aminés : LLFLLLADA (SEQ ID NO : 2) ; la complexation du peptide immunogène avec une molécule A2.1 HLA ; et la mise en contact in vitro d'un CTL restreint par A2.1 HLA avec le complexe du peptide et la molécule A2.1 HLA, moyennant quoi une réponse CTL est induite.

16. Méthode selon la revendication 15, dans laquelle le peptide consiste en la séquence d'acides aminés : LLFLLLADA (SEQ ID NO : 2).

17. Utilisation d'un peptide immunogène pour la fabrication d'un médicament pour le traitement ou la prévention d'une infection par le VHC, dans laquelle le peptide est inférieur à 15 acides aminés et comprend la séquence d'acides LLFLLLADA (SEQ ID NO : 2) .

18. Utilisation selon la revendication 17, dans laquelle le peptide consiste en la séquence d'acides aminés LLFLLLADA (SEQ ID NO : 2).

19. Utilisation d'un acide nucléique pour la fabrication d'un médicament pour le traitement ou la prévention d'une infection par le VHC, dans laquelle l'acide nucléique comprend une séquence de nucléotides codant pour un polypeptide qui comprend des épitopes de CTL provenant du VHC joints les uns aux autres, dans laquelle un ou plusieurs épitopes comprend (comprennent) la séquence d'acides aminés LLFLLLADA (SEQ ID NO : 2).

20. Utilisation selon la revendication 19, dans laquelle l'un des épitopes consiste en la séquence d'acides aminés LLFLLLADA (SEQ ID NO : 2).

21. Utilisation selon la revendication 19, dans laquelle le polypeptide comprend d'autres épitopes consistant en les séquences d'acides aminés : ILSPGALVV (SEQ ID NO : 1), LLFLLLADA (SEQ ID NO : 2), WMNRLIAFA (SEQ ID NO : 3), et VLVGGVLAA (SEQ ID NO : 4).

22. Utilisation selon l'une quelconque des revendications 19 à 21, dans laquelle le polypeptide comprend des séquences flanquantes, adjacentes à un ou plusieurs des épitopes de CTL.

23. Méthode selon la revendication 15 ou 16, dans laquelle la molécule A2.1 HLA est exprimée sur une cellule.


### SEQUENCE LISTING

<110> Epimmune, Inc
<120> HLA-A2.1 Binding Peptides and their Uses
<130> WA/47565
<140> EP 05075377.1
   <141> 1997-03-21
<150> US 60/013,980
   <151> 1996-03-21
<150> US 08/822,382
   <151> 1997-03-20
<150> EP 97918494.2
   <151> 1997-03-21
<160> 44
<170> Patent In version 3.1
<210> 1
   <211> 9
   <212> PRT
   <213> Hepatitis C Virus
<400> 1
   Ile Leu Ser Pro Gly Ala Leu Val Val
   1               5
<210> 2
   <211> 9
   <212> PRT
   <213> Hepatitis C Virus
<400> 2
   Leu Leu Phe Leu Leu Leu Ala Asp Ala
   1 5
<210> 3
   <211> 9
   <212> PRT
   <213> Hepatitis C Virus
<400> 3
   Trp Met Asn Arg Leu Ile Ala Phe Ala
   1 5
<210> 4
   <211> 9
   <212> PRT
   <213> Hepatitis C Virus
<400> 4
   Val Leu Val Gly Gly Val Leu Ala Ala
   1 5
<210> 5
   <211> 9
   <212> PRT
   <213> Human immunodeficiency virus type 1
<400> 5
   Leu Leu Gln Leu Thr Val Trp Gly Ile
   1 5
<210> 6
   <211> 9
   <212> PRT
   <213> Human immunodeficiency virus type 1
<400> 6
   Met Thr Asn Asn Pro Pro Ile Pro Val
   1 5
<210> 7
   <211> 9
   <212> PRT
   <213> Human immunodeficiency virus type 1
<400> 7
   Phe Leu Gln Ser Arg Pro Glu Pro Thr
   1 5
<210> 8
   <211> 9
   <212> PRT
   <213> Human immunodeficiency virus type 1
<400> 8
   Leu Thr Phe Gly Trp Cys Phe Lys Leu
   1 5
<210> 9
   <211> 9
   <212> PRT
   <213> Human immunodeficiency virus type 1
<400> 9
   Thr Leu Asn Phe Pro Ile Ser Pro Ile
   1 5
<210> 10
   <211> 9
   <212> PRT
   <213> Human immunodeficiency virus type 1
<400> 10
   Tyr Thr Ala Phe Thr Ile Pro Ser Ile
   1 5
<210> 11
   <211> 9
   <212> PRT
   <213> Human immunodeficiency virus type 1
<400> 11
   Thr Leu Trp Gln Arg Pro Leu Val Thr
   1 5
<210> 12
   <211> 9
   <212> PRT
   <213> Human immunodeficiency virus type 1
<400> 12
   Lys Ala Ala Cys Trp Trp Ala Gly Ile
   1 5
<210> 13
   <211> 9
   <212> PRT
   <213> Human immunodeficiency virus type 1
<400> 13
   Ala Leu Val Glu Ile Cys Thr Glu Met
   1 5
<210> 14
   <211> 9
   <212> PRT
   <213> Human immunodeficiency virus type 1
<400> 14
   Leu Val Gly Pro Thr Pro Val Asn Ile
   1 5
<210> 15
   <211> 9
   <212> PRT
   <213> Human immunodeficiency virus type 1
<400> 15
   Arg Ala Met Ala Ser Asp Phe Asn Leu
   1 5
<210> 16
   <211> 9
   <212> PRT
   <213> Human immunodeficiency virus type 1
<400> 16
   Gln Leu Leu Phe Ile His Phe Arg Ile
   1 5
<210> 17
   <211> 9
   <212> PRT
   <213> Human immunodeficiency virus type 1
<400> 17
   Ala Ile Ile Arg Ile Leu Gln Gln Leu
   1 5
<210> 18
   <211> 9
   <212> PRT
   <213> Human immunodeficiency virus type 1
<400> 18
   Arg Ile Leu Gln Gln Leu Leu Phe Ile
   1 5
<210> 19
   <211> 9
   <212> PRT
   <213> Human immunodeficiency virus type 1
<400> 19
   Lys Leu Val Gly Lys Leu Asn Trp Ala
   1 5
<210> 20
   <211> 10
   <212> PRT
   <213> Human immunodeficiency virus type 1
<400> 20
   Trp Met Thr Asn Asn Pro Pro Ile Pro Val
   1 5 10
<210> 21
   <211> 10
   <212> PRT
   <213> Human immunodeficiency virus type 1
<400> 21
   Pro Leu Thr Phe Gly Trp Cys Phe Lys Leu
   1 5 10
<210> 22
   <211> 10
   <212> PRT
   <213> Human immunodeficiency virus type 1
<400> 22
   Leu Thr Phe Gly Trp Cys Phe Lys Leu Val
   1 5 10
<210> 23
   <211> 10
   <212> PRT
   <213> Human immunodeficiency virus type 1
<400> 23
   Met Ala Ser Asp Phe Asn Leu Pro Pro Val
   1 5 10
<210> 24
   <211> 10
   <212> PRT
   <213> Human immunodeficiency virus type 1
<400> 24
   Cys Thr Leu Asn Phe Pro Ile Ser Pro Ile
   1               5                   10
<210> 25
   <211> 10
   <212> PRT
   <213> Human immunodeficiency virus type 1
<400> 25
   Lys Leu Asn Trp Ala Ser Gln Ile Tyr Ala
   1               5                   10
<210> 26
   <211> 10
   <212> PRT
   <213> Human immunodeficiency virus type 1
<400> 26
   Lys Met Ile Gly Gly Ile Gly Gly Phe Ile
   1               5                   10
<210> 27
   <211> 10
   <212> PRT
   <213> Hepatitis B Virus
<220>
   <221> misc_feature
   <222> (5)..(5)
   <223> Xaa = biotinylated Lys
<400> 27
   Phe Leu Pro Ser Xaa Tyr Phe Pro Ser Val
   1               5                   10
<210> 28
   <211> 10
   <212> PRT
   <213> Hepatitis B Virus
<220>
   <221> MISC_FEATURE
   <222> (6)..(6)
   <223> X = biotinylated Lys
<400> 28
   Phe Leu Pro Ser Asp Xaa Phe Pro Ser Val
   1 5 10
<210> 29
   <211> 9
   <212> PRT
   <213> Hepatitis B Virus
<400> 29
   Tyr Met Asp Asp Val Val Leu Gly Val
   1 5
<210> 30
   <211> 10
   <212> PRT
   <213> Hepatitis B Virus
<400> 30
   Phe Leu Pro Ser Glx Phe Phe Pro Ser Val
   1 5 10
<210> 31
   <211> 10
   <212> PRT
   <213> Hepatitis B Virus
<400> 31
   Phe Leu Pro Ser Asp Ala Phe Pro Ser Val
   1 5 10
<210> 32
   <211> 10
   <212> PRT
   <213> Hepatitis B Virus
<400> 32
   Phe Leu Pro Ser Ala Phe Phe Pro Ser Val
   1 5 10
<210> 33
   <211> 10
   <212> PRT
   <213> Hepatitis B Virus
<400> 33
   Phe Leu Pro Ser Asp Phe Ala Pro Ser Val
   1 5 10
<210> 34
   <211> 10
   <212> PRT
   <213> Hepatitis B Virus
<400> 34
   Phe Leu Pro Ser Asp Phe Phe Ala Ser Val
   1 5 10
<210> 35
   <211> 10
   <212> PRT
   <213> Hepatitis B Virus
<400> 35
   Phe Leu Pro Ser Asp Phe Phe Pro Ala Val
   1 5 10
<210> 36
   <211> 10
   <212> PRT
   <213> Hepatitis B Virus
<400> 36
   Phe Leu Ala Ser Asp Phe Phe Pro Ser Val
   1 5 10
<210> 37
   <211> 10
   <212> PRT
   <213> Hepatitis B Virus
<400> 37
   Ala Leu Pro Ser Asp Phe Phe Pro Ser Val
   1 5 10
<210> 38
   <211> 10
   <212> PRT
   <213> Hepatitis B Virus
<400> 38
   Tyr Leu Pro Ser Asp Phe Phe Pro Ser Val
   1 5 10
<210> 39
   <211> 10
   <212> PRT
   <213> Hepatitis B Virus
<400> 39
   Phe Met Pro Ser Asp Phe Phe Pro Ser Val
   1 5 10
<210> 40
   <211> 10
   <212> PRT
   <213> Hepatitis B Virus
<400> 40
   Phe Leu Lys Ser Asp Phe Phe Pro Ser Val
   1 5 10
<210> 41
   <211> 10
   <212> PRT
   <213> Hepatitis B Virus
<400> 41
   Phe Leu Pro Ser Glu Phe Phe Pro Ser Val
   1 5 10
<210> 42
   <211> 10
   <212> PRT
   <213> Hepatitis B Virus
<400> 42
   Phe Leu Pro Ser Asp Phe Tyr Pro Ser Val
   1 5 10
<210> 43
   <211> 10
   <212> PRT
   <213> Hepatitis B Virus
<400> 43
   Phe Leu Pro Ser Asp Phe Phe Lys Ser Val
   1 5 10
<210> 44
   <211> 10
   <212> PRT
   <213> Hepatitis B Virus
<400> 44
   Phe Leu Pro Ser Asp Phe Phe Pro Lys Val
   1 5 10